(19) Europäisches Patentamt
European Patent Office
Office européen des brevets

(11) Publication number: 0 236 533 A1

# (12) EUROPEAN PATENT APPLICATION

(21) Application number: 86103480.9

(22) Date of filing: 14.03.86

(51) Int. Cl.4: A61K 7/09

(43) Date of publication of application: 16.09.87 Bulletin 87/38

(84) Designated Contracting States: BE DE FR GB IT NL

(71) Applicant: SUMITOMO PHARMACEUTICALS COMPANY, LIMITED
40, Dosho-machi 2-chome
Higashi-ku Osaka-shi Osaka-fu(JP)
Applicant: Yamahatsu Sangyo Kaisha Ltd.
No. 1-25, Dozima 1-chome Kita-ku
Osaka-shi Osaka-fu(JP)

(72) Inventor: Matsumoto, Heiichiro
No. 1345-2, Kujyocho
Yamatokoriyama-shi Nara-ken(JP)
Inventor: Nakao, Masaru
No. 1-4-26, Aioidori Abeno-ku
Osaka-shi Osaka-fu(JP)

(74) Representative: Vossius & Partner
Siebertstrasse 4 P.O. Box 86 07 67
D-8000 München 86(DE)

## (54) Permanent wave preparation.

(57) A permanent wave first preparation comprising as an active ingredient homocysteine or its ester and, preferably, an organic weak base and/or thioglycolic acid or its salt.

EP 0 236 533 A1

# PERMANENT WAVE PREPARATION

The present invention relates to a permanent wave preparation. More particularly, it relates to a permanent wave first preparation.

Generally, two preparations are used for having a permanent waving. One is a permanent wave first preparation which is a liquid preparation containing as a main ingredient a reducing agent and the other is a permanent wave second preparation which is a liquid preparation containing as a main ingredient an oxidant such as a bromate, a perborate, etc. In order to have a person's hair permed, firstly, a permanent wave first preparation is applied to the hair to reductively cleave the disulfide bonds (-S-S-) in the hair protein and then the hair is curled in a desired shape to wave the hair. After waving, the hair is treated with a permanent wave second preparation to oxidatively regenerate the disulfide bonds in the hair protein to set the waves.

Heretofore, thioglycolic acid have been widely used as a reducing agent of a permanent wave first preparation because is has excellent wave forming ability and wave permanence. However, there are many problems in the use of thioglycolic acid. For example, thioglycolic acid has bad odor. Further, because of its violent activity, thioglycolic acid impairs gloss and elasticity of a hair to injure the hair and, in some cases, it causes skin disorders due to its stimulation.

In order to solve these problems, recently, a permanent wave first preparation using cysteine instead of thioglycolic acid as a reducing agent has been developed and practically used. This permanent wave first preparation is superior to that using thioglycolic acid in respect of odor and stimulation. However, there are also problems in a permanent wave first preparation using cysteine. For example, wave forming ability of a first preparation using cysteine is lower than that using thioglycolic acid. Further, in a first preparation using cysteine, cystine derived from cysteine is crystallized out during using the preparation to have a permanent, which results in so-called "flaking" on a customer's hair and user's hands to hurt their feelings.

The present inventors have intensively studied to obtain a permanent wave first preparation which can exhibit excellent properties without above problems of conventional permanent wave first preparations. As the result, it has been found that homocysteine or its ester is suitable for using as a reducing agent of a permanent wave first preparation. Further, it has been also found that when homocysteine or its ester is used together with an organic base and/or a relatively small amount of thioglycolic acid, properties of a permanent wave first preparation can be further improved.

One object of the present invention is to provide a novel permanent wave first preparation which can exhibit excellent wave forming ability and wave permanence preventing bad odor, injury of the hair and stimulation.

Another object of the present invention is to provide a novel permanent wave first preparation causing less flaking.

According to the present invention, there is provided a permanent wave first preparation comprising as an active ingredient homocysteine or its ester of the formula:

$$HS-CH_2-CH_2-\underset{\displaystyle NH_2}{\underset{|}{C}H}-COOR \qquad [I]$$

wherein R is hydrogen atom or a lower alkyl group. In a preferred aspect of the present invention, the permanent wave first preparation further comprises an organic base and/or thioglycolic acid.

When homocysteine or its ester is used in a permanent wave first preparation, the resulting preparation has much less odor than that of a conventional preparation using thioglycolic acid and is safer because of less injury of the hair and stimulation. Further, it has been found that wave forming ability of homocysteine is higher than that of cysteine. In addition, although homocystine is also crystallized out from homocysteine during the use of the preparation, the degree of crystallization of homocystine from homocysteine is lower thant that of cystine from cysteine.

On the other hand, homocysteine has somewhat low shelf stability. Thereofe, in the preferred aspect of the present invention, an organic base and/or thioglycolic acid in an amount that it does not adversely affect odor and safety are used together with homocysteine or its ester, and, thereby, a permanent wave first preparation having improved wave permanence can be obtained without causing any problem in odor, injury of the hair and safety, wave forming ability, flaking and shelf stability. Particularly, the use of an organic base together with homocysteine or its ester improves wave forming ability, wave permanence, inhibition of crystallization, shelf stability and solubility, and the use of thioglycolic acid together with homocysteine or its ester improves wave permanence and shelf stability. In the present invention, it is preferable to use both an organic base and thioglycolic acid together with homocysteine or its ester.

In homocysteine or its ester of the above formula [I], the alkyl group represented by R includes that having 1 to 6 carbon atoms such as methyl, ethyl and the like. Homocysteine can be economically produced from methionine, which is commercially available as medicine and feed additive, in an industrial scale according to a method described in, for example, Biochemical Preparations, Vol. 5, pages 91-96 - (1957). Its ester can be produced according to a conventional esterification. As is seen from the formula [I], homocysteine or its ester has an amino group and it can form a salt with an inorganic acid such as hydrochloride and the like. Further, when R is a hydrogen atom, a carboxyl group is present and it can form a salt with an inorganic or organic base such as sodium salt, potassium salt, ammonium salt, monoethanolamine salt and the like. These salts can be used in a similar manner as homocysteine or its ester and their use also falls within the scope of the claims described hereinafter. In the present invention, from the view point of properties of the permanent wave first preparation to be obtained, usually, homocysteine or its ester is used in an amount of 2 to 10% by weight, preferably, 3 to 6% by weight based on the total weight of the preparation.

Suitably, the organic base used in the present invention is a non-volatile weak organic base and includes monoethanolamine, diethanolamine, triethanolamine, aminomethyl propanediol, isopropylamine and the like. A single organic base or a combination thereof can be used. Particularly, from the viewpoint of inhibition of flaking, monoethanolamine is preferred. Although the above organic base has been known as an ingredient for improving properties of a reducing agent in a conventional permanent wave first preparation, in the present invention, it is used for improving solubility of homocysteine to inhibit crystallizing out, in addition to improving properties of the reducing agent, i.e., homocysteine. Generally, the organic base is used in an amount of 0.5 to 6% by weight, preferably, 2.0 to 4.0% by weight based on the total weight of the preparation. When the amount of the organic base is less than this range, the effect thereof is hardly expected. On the other hand, when the amount of the organic base exceeds the above range, it tends to cause stimulation of the skin and injury of the hair.

The thioglycolic acid may be used as a salt such as ammonium thioglycolate, monoethanolamine thioglycolate, and the like. For example, commercially available 50% aqueous solution of ammonium thioglycolate and 30 to 40% aqueous solution of monoethanolamine thioglycolate can be used. Thioglycolic acid or its salt is used in an amount of 0.05 to 3% by weight, preferably, 0.2 to 2% by weight, more preferably, 0.5 to 1.5% by weight calculated as its free acid based on the total weight of the preparation. Apparently, when the amount of thioglycolic acid is increased, it adversely affects odor of the preparation and causes injury of the hair and safety problems. Therefore, in the present invention, it is preferable to use thioglycolic acid in an amount as small as possible, so long as the effect on the improvement of shelf stability can be obtained.

Basically, the permanent wave first preparation of the present invention is a liquid preparation comprising homocysteine or its salt and other ingredients, if any, dispersed or dissolved in water. Preferably, the preparation contains either the organic base or thioglycolic acid, or both the organic base and thioglycolic acid. Optionally, the preparation of the present invention can further contain conventional additives such as bases other than the above for adjusting pH, surfactants, fats and oils, colorants, perfumes, moisturizing agents and the like.

The permanent wave first preparation of the present invention can be produced according to a standard method such as mixing, dispersing and dissolving the desired ingredients. By the way, in the production of the permanent wave first preparation of the present invention, deaeration of water to be used, replacement of dissolved oxygen with nitrogen and/or nitrogen flush packaging can be effected according to a known method to further improve shelf stability.

The permanent wave first preparation of the present invention can be used in a similar manner as a conventional permanent wave first preparation to have a person's hair permed. That is, it can be applied to any permanent waving technique such as cold wave technique, warming technique or barber's curling iron technique and exhibits improved wave forming ability, wave permanence and shelf stability with preventing problems in odor, injury of a hair, safety, flaking, and the like.

The following Examples and Comparative Examples further illustrate the present invention in detail, but are not to be construed to limit the scope thereof.

Example 1

| Ingredients | Amounts |
|---|---|
| Homocysteine | 4.0 g |
| Monoethanolamine | 3.0 g |
| Polyoxyethylene oleyl ether (7 E.O.) | 0.5 g |
| Polyoxyethylene oleyl ether (20 E.O.) | 0.8 g |
| Sodium lauryl sulfate | 0.4 g |
| Cationic cellulose | 0.1 g |
| Disodium edetate | 5.1 g |
| Colorant | q.p. |
| Perfume | q.p. |
| Distilled water | up to 100 ml |

About one half of the distilled water was warmed to 40°C and sodium lauryl sulfate was dissolved in the warmed water. Then, homocysteine, monoethanolamine, polyoxyethylene oleyl ethers, cationic cellulose, disodium edetate, colorant and perfume were successively added to the solution and the remaining distilled water was added thereto to adjust the total volume to 100 ml.

Examples 2 to 8 and Comparative Examples 1 and 2

According to the formulation shown in Table 1 hereinafter, each permanent wave first preparation (100 ml) was produced by the same manner as in Example 1.

Each preparation thus obtained was tested for permanent wave effect, degree of flaking, degree of hair injury, odor, skin stimulation and shelf stability as follows.

(1) Permanent wave effect

Permanent wave effect of the preparation was tested according to Kirby method described in "Hair and Perm" edited by Japanese Permanent Wave Solutions Industrial Association, pages 99 to 100 (1980).

That is, a human hair (1 g) was thoroughly washed with water and air-dried. The hair was waved on a plate for measuring wave degree and fixed on the plate. The plate was dipped in a bath containing a sample of each permanent wave first preparation (90 ml) maintained at 35°C for 20 minutes. Then, the plate was taken out of the bath and washed with water. The plate thus treated was again dipped in another bath containing 6% aqueous solution of sodium bromate as a permanent wave second preparation which was maintained at 35°C for 15 minutes. Then, the plate was taken out of the bath and thoroughly washed with water. After these treatments, the hair was removed from the plate and placed on a glass plate to determine the wavelength of the waved hair and record wave efficiency immediately after treatment (a). Then, the hair was dipped in 7.5% aqueous solsution of sodium polyoxyethylene lauryl ether sulfate (2 E.O.) maintained at 60°C for 1 hour. After dipping, likewise, the wavelength of the hair was determined to record wave efficiency (b). By using these values, a wave retention ration (b/a) was calculated. A larger retention ratio represents better wave permanence.

(2) Degree of flaking

A sample of each permanent wave first preparation was applied to a bundle of hair (3 g) and allowed to stand at room temperature for 30 minutes. A degree of crystallization was observed by the naked eye and evaluated according to the following criteria.
+: much crystallization
±: slight crystallization
-: no crystallization

(3) Degree of hair injury

According to the same manner as in the above test (1), a bundle of hair was waved and allowed to stand to dry it. Then, the weight of the bundle (c) was determined and the bundle was dipped in distilled water (50 ml) for 1 hour. After taking out the bundle, water adhered to the surface of the hair was completely removed and the weight of the bundle (d) was again determined. By using these values, water absorption ratio was calculated according to the following formula:

$$\text{Water absorption ratio (\%)} = \frac{d - c}{c} \times 100$$

A higher water absorption ratio represents a higher degree of injury of the hair.

(4) Odor

By applying each preparation to a hair, the resulting reaction odor was organoleptically evaluated according to the following criteria.
A: no odor
B: slight odor
C: bad odor

(5) Skin stimulation

A patch test was carried out applying lint impregnated with a sample of each permanent wave first preparation (0.5 ml) to the shaved back of a rabbit for 4 hours. The topical reaction of the applied part was observed and the skin stimulation was evaluated according to the following criteria.
A: no stimulation
B: slight stimulation
C: strong stimulation

(6) Shelf stability

Immediately after production of each preparation, the homocysteine content was determined by iodometry. Likewise, after storage for 50 days, the homocysteine content of the preparation was determined. A relative ratio of the content after storage for 50 days to that of immediately after production was calculated by taking the latter as 100.
The results are also shown in Table 1.

Table 1

| Ingredients | Example Nos. 2 | 3 | 4 | 5 | 6 | 7 | 8 | Compar. Ex. Nos. 1 | 2* |
|---|---|---|---|---|---|---|---|---|---|
| Homocysteine (g) | 5.0 | 5.0 | 5.0 | 5.0 | 5.0 | 5.0 | 5.0 | 5.0 | 6.0 |
| Monoethanolamine (g) | 3.0 | 3.0 | 3.0 | 3.0 | 3.0 | 3.0 | 3.0 | 3.0 | 4.5 |
| Ammonium thioglycolate 50% aqueous solution (g)** | - - | 0.4 (0.2) | 1.0 (0.5) | 1.6 (0.8) | 2.2 (1.1) | 2.8 (1.4) | 4.0 (2.0) | 8.0 (4.0) | - - |
| Polyoxyethylene oleyl ether (7 E.O.) (g) | 0.5 | 0.5 | 0.5 | 0.5 | 0.5 | 0.5 | 0.5 | 0.5 | 0.5 |
| Polyoxyethylene oleyl ether (20 E.O.) (g) | 0.8 | 0.8 | 0.8 | 0.8 | 0.8 | 0.8 | 0.8 | 0.8 | 0.8 |
| Sodium lauryl sulfate (g) | 0.4 | 0.4 | 0.4 | 0.4 | 0.4 | 0.4 | 0.4 | 0.4 | 0.4 |
| Distilled water | to 100 ml | to 100 ml | to 100 ml | to 100 ml | to 100 ml | to 100 ml | to 100 ml | to 100 ml | to 100 ml |
| Permanent wave effect | | | | | | | | | |
| a (%) | 67.79 | 70.47 | 72.48 | 73.15 | 77.85 | 79.87 | 81.21 | 82.55 | 44.97 |
| b (%) | 44.30 | 53.36 | 64.43 | 58.39 | 70.47 | 69.80 | 69.80 | 69.80 | 24.83 |
| b/a | 65.3 | 77.1 | 88.9 | 79.8 | 90.5 | 87.4 | 86.0 | 84.6 | 55.2 |
| Degree of flaking | ± | ± | - | - | - | - | - | - | ± |
| Degree of hair injury*** | 26.8 | - | 27.0 | - | 27.5 | - | 28.3 | 38.8 | - |
| Odor | A | A | A | A | A | B | B | C | A |
| Skin stimulation | A | A | A | A | A | A | B | C | A |
| Shelf stability | 93.0 | - | 98.8 | - | 99.4 | - | 99.6 | 99.7 | - |

Notes of Table 1:

*: Instead of homocysteine, cysteine was used.

**: The number in the parentheses represents amount of ammonium thioglycolate itself.

***: The water absorption ratio of a hair without permanent wave treatment is 26.0.

As shown in Table 1, the permanent wave first preparation of the present invention has excellent properties.

## Claims

1. A permanent wave first preparation comprising as an active ingredient homocysteine or its ester of the formula:

$$HS-CH_2-CH_2-\underset{\substack{|\\ NH_2}}{CH}-COOR$$

wherein R is a hydrogen atom or a lower alkyl group.

2. A permanent wave first preparation according to claim 1, wherein the preparation further comprises an organic base and/or thioglycolic acid or its salt.

3. A permanent wave first preparation according to claim 2, wherein the organic base is monoethanolamine, diethanolamine, triethanolamine, aminomethyl propanediol or isopropylamine.

4. A permanent wave first preparation according to claim 1, wherein the preparation contains the homocysteine or its ester in an amount of 2 to 10% by weight based on the total weight of the preparation.

5. A permanent wave first preparation according to claim 2, wherein the preparation contains the organic base in an amount of 0.5 to 6% by weight based on the total weight of the preparation.

6. A permanent wave first preparation according to claim 2, wherein the preparation contains thioglycolic acid or its salt in an amount of 0.05 to 3% by weight as calculated as free acid based on the total weight of the preparation.

European Patent Office

# EUROPEAN SEARCH REPORT

Application number

EP 86 10 3480

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl 4) |
| Y | FR-A-2 133 991 (L'OREAL)<br>* Whole document * | 1,2,4-6 | A 61 K 7/09 |
| Y | FR-A-2 092 822 (BRISTOL-MYERS)<br>* Page 4, line 25 - page 7, line 5; page 7, lines 6-16; claims 1-10 * | 1,2,4-6 | |
| E | PATENTS ABSTRACTS OF JAPAN, vol. 10, no. 235 (C-366)[2294], 14th August 1986; & JP-A-61 69 715 (SUMITOMO CHEM. CO. LTD.) 10-04-1986 | 1,4 | |
| A | FR-A-1 233 523 (DEUTSCHE GOLD UND SILBER-SCHEIDEANSTALT)<br>* Whole document * | 1,2,4 | TECHNICAL FIELDS SEARCHED (Int. Cl 4)<br>A 61 K |
| A | LU-A- 53 453 (UNILEVER)<br>* Page 2, line 12 - page 4, line 18; claims 1,5-16 * | 1-5 | |
| A | GB-A- 797 167 (DEVON COLD WAVE CO.)<br>* Whole document * | 1,4 | |
| | --- -/- | | |

The present search report has been drawn up for all claims

| Place of search | Date of completion of the search | Examiner |
|---|---|---|
| THE HAGUE | 07-10-1986 | FISCHER J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 86 10 3480



**DOCUMENTS CONSIDERED TO BE RELEVANT**

| Category | Citation of document with indication, where appropriate, of relevant passages | Relevant to claim | CLASSIFICATION OF THE APPLICATION (Int. Cl 4) |
|---|---|---|---|
| A | PATENTS ABSTRACTS OF JAPAN, vol. 5, no. 148 (C-72)[820], 18th September 1981; & JP-A-56 79 618 (TANABE SEIYAKU K.K.) 30-06-1981 | 1,2,4, 6 | |
| | --- | | |
| A | DE-A-1 959 149 (THE GILLETTE CO.) * Page 10, lines 9-19; claims 1-4 * | 1 | |
| | ----- | | |
| | | | TECHNICAL FIELDS SEARCHED (Int. Cl 4) |

The present search report has been drawn up for all claims

| Place of search | Date of completion of the search | Examiner |
|---|---|---|
| THE HAGUE | 07-10-1986 | FISCHER J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82